(19) Europäisches Patentamt
European Patent Office
Office européen des brevets

(11) **EP 4 216 227 A1**

(12) **EUROPEAN PATENT APPLICATION**

(43) Date of publication:
**26.07.2023 Bulletin 2023/30**

(21) Application number: **23152848.0**

(22) Date of filing: **23.01.2023**

(51) International Patent Classification (IPC):
**G16H 20/17** (2018.01)    **G16H 40/63** (2018.01)
**G06F 3/04847** (2022.01)

(52) Cooperative Patent Classification (CPC):
**G16H 20/17; G06F 3/04847; G16H 40/63**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.01.2022 US 202263302274 P**

(71) Applicant: **Insulet Corporation**
**Acton MA 01720 (US)**

(72) Inventors:
• **LEE, Joon Bok**
  **Acton (US)**
• **O'CONNOR, Jason**
  **Acton (US)**
• **ZHENG, Yibin**
  **Hartland (US)**
• **NELSON, Graeme**
  **Oakland (US)**

(74) Representative: **Peterreins Schley**
**Patent- und Rechtsanwälte PartG mbB**
**Hermann-Sack-Straße 3**
**80331 München (DE)**

(54) **TECHNIQUES AND SYSTEM FOR PARAMETER SELECTION FOR ONBOARDING AND ONGOING MANAGEMENT OF PUMP USERS**

(57)    Disclosed are a computing apparatus, a computer-readable medium and a method that enable a user to use subjective inputs to alter settings of a wearable automatic drug delivery system. A user input device is presented on a graphical user interface that enables input of a subjective insulin need parameter. In response to receiving the input, the processor may modify a subjective coefficient value. A specific factor useable by an automatic insulin delivery algorithm is set based on the subjective coefficient value. Physiological condition data related to the user and the set specific factor may be used to determine a dosage of insulin to be delivered to the user based on the collected physiological condition data of the user. The processor may cause the determined dosage of insulin to be delivered to the user based on an output of the automatic insulin delivery algorithm.

FIG. 1A

EP 4 216 227 A1

**Description**

**BACKGROUND**

The use of insulin pumps for people with Type 1 Diabetes Mellitus typically require users to provide a wide range of complex and technical parameters to initiate insulin therapy via an automatic insulin delivery (AID) algorithm. The complexity of the calculations performed by the AID algorithms using the clinical parameters may make new users as well as some healthcare providers weary of adjusting the parameter settings, as such, the parameter settings of the AID algorithms may not provide an optimal outcome for the user. As a result, entry of these parameters can become a source of significant risk for user error as well as an inconvenience that serves as a barrier to increasing the use of insulin pumps. Furthermore, as personal conditions change over time, maintaining these parameters is a source of further inconvenience for the user.

One method to address these inconveniences is by requesting users to provide a single demographic parameter that is easily understandable and well known - such as the user's weight - as part of the onboarding process. However, users with the same demographics (e.g, same weight) may have significantly different insulin sensitivities, and insulin therapy based on one demographic parameter may not provide safe and effective glucose control outcomes for all of the users with the same demographic parameter.

Therefore, it would be beneficial and advantageous if a user when onboarding or even during use, could simply set parameters of their AID algorithm through easy to use, subjective user inputs.

**SUMMARY**

In one aspect, a computing apparatus includes a processor, a user interface and a memory storing instructions and an automatic insulin delivery algorithm. The computing apparatus, when the processor is executing the instructions and the automatic insulin delivery algorithm, is operable to present a user input device on a graphical user interface of the user interface. The graphical user interface offers an input device that enables input and receiving via the input device a subjective insulin need parameter. In response to receiving the input of the subjective insulin need parameter, the processor may modify a subjective coefficient value. One or more of a number of specific factors useable by an automatic insulin delivery algorithm may be set based on the subjective coefficient value. Physiological condition data related to the user may be collected. A dosage of insulin to be delivered to the user may be determined using the one or more of the number of specific factors set based on the subjective coefficient value and based on the collected physiological condition data of the user. The processor may cause the determined dosage of insulin to be delivered to the user in response to an output of the automatic insulin delivery algorithm.

In another aspect, a method is provided that includes presenting, on a user interface of a user device, a graphical user interface offering an input device that enables input of a subjective insulin need parameter. The input of the subjective insulin need parameter is received via the input device. In response to receiving the input of the subjective insulin need parameter, a subjective coefficient value may be modified. One or more of a number of specific factors useable by an automatic insulin delivery algorithm may be set based on the subjective coefficient value. The collection of physiological condition data related to the user may begin. A dosage of insulin to be delivered to the user using the one or more of the number of specific factors may be determined based on the subjective coefficient value and on the collected physiological condition data of the user. The determined dosage of insulin may be caused to be delivered to the user in response to an output of the automatic insulin delivery algorithm.

In a further aspect, a non-transitory computer-readable storage medium is provided. The computer-readable storage medium may include instructions that when executed by a processor, cause the processor to present, on a user interface of a user device, a graphical user interface offering an input device that enables input of a subjective insulin need parameter. The input of the subjective insulin need parameter may be received via the input device. In response to receiving the input of the subjective insulin need parameter, a subjective coefficient value may be modified. One or more of a number of specific factors useable by an automatic insulin delivery algorithm may be set based on the subjective coefficient value. Collection of physiological condition data related to the user may begin. A dosage of insulin to be delivered to the user may be determined based on the collected physiological condition data of the user and using one or more of the number of specific factors set based on the subjective coefficient value. The determined dosage of insulin may be caused to be delivered to the user in response to an output of the automatic insulin delivery algorithm.

**BRIEF DESCRIPTION OF THE DRAWINGS**

In the drawings, like reference characters generally refer to the same parts throughout the different views. In the following description, various embodiments of the present disclosure are described with reference to the following drawings, in which:

FIG. 1A illustrates an example of a graphical user interface for input of a subjective insulin need parameter according to an embodiment of the disclosed subject matter.

FIG. 1B illustrates an explanation of the input entered in the example of the graphical user interface according to an embodiment of the disclosed subject matter.

FIG. 2 illustrates an example for determining a value of a subjective coefficient from a user input in accordance with one embodiment.

FIG. 3 is a flowchart illustrating a routine in accordance with an embodiment.

FIG. 4 illustrates a graph illustrating an example for determining a value of a subjective coefficient from a user input in accordance with multiple embodiments.

FIG. 5 illustrates a graph illustrating another example for determining a value of a subjective coefficient from a user input in accordance with multiple embodiments.

FIG. 6 illustrates a graph illustrating a further example for determining a value of a subjective coefficient from a user input in accordance with multiple embodiments.

FIG. 7 illustrates a graph illustrating yet another example for determining a value of a subjective coefficient from a user input in accordance with multiple embodiments.

FIG. 8 illustrates a drug delivery system operable to implement the examples described with reference to FIGs. 1A-7.

## DETAILED DESCRIPTION

Systems, devices, computer-readable medium and methods in accordance with the present disclosure will now be described more fully hereinafter with reference to the accompanying drawings, where one or more embodiments are shown. The systems, devices, and methods may be embodied in many different forms and are not to be construed as being limited to the embodiments set forth herein. Instead, these embodiments are provided so the disclosure is thorough and complete, and fully conveys the scope of methods and devices to those skilled in the art. Each of the systems, devices, and methods disclosed herein provides one or more advantages over conventional systems, components, and methods.

The disclosed examples provide techniques that may be used with any additional algorithms or computer applications that manage blood glucose levels, diabetes therapy, and/or other therapeutic programs. These algorithms and computer applications may be collectively referred to as "medication delivery algorithms" or "medication delivery applications" and may be operable to deliver different categories of drugs (or medications), such as chemotherapy drugs, pain relief drugs, diabetes treatment drugs (e.g., insulin, glucagon and/or glucagon-like peptides), blood pressure medication, or the like.

A type of medication delivery algorithm (MDA) may include an "artificial pancreas" algorithm-based system, or more generally, an artificial pancreas (AP) application. For ease of discussion, the computer programs and computer applications that implement the medication delivery algorithms or applications may be referred to herein as an "AID algorithm." An AID algorithm may be configured to provide automatic delivery of insulin based on an analyte sensor input, such as signals received from an analyte sensor, such as a continuous blood glucose monitor, or the like.

In contrast to systems that allow input of a single demographic parameter, such as weight, gender, age or the like, the disclosed AID algorithm or AID application when executed by a processor may enable a user to input a subjective parameter related to their drug treatment program (i.e., dosage amounts, dosage delivery timing, system-generated recommendations, and the like). In the following examples, the AID algorithm may utilize the inputted subjective parameter to establish settings within the AID algorithm for the user that are intended to optimize the user's drug treatment program. The AID algorithm enables the wearable drug delivery system to monitor a user's blood glucose measurement values, determine an appropriate level of insulin for the user based on the monitored glucose values (e.g., blood glucose concentrations or blood glucose measurement values) and other information, such as information related to, for example, a correction factor or substance sensitivity factor, and take actions to maintain a user's blood glucose value within an appropriate range.

Various examples provide a method, a system, a device and a computer-readable medium for responding to inputs provided by sensors, such as an analyte sensor, and users of an automatic drug delivery system. "Analyte sensor" may refer to a wearable device that includes sensing and measurement devices configured to detect different analytes, such as blood glucose, in a user's body and particularly in the user's blood, as well as other analytes as described herein. The various devices and sensors that may be used to implement some specific examples may also be used to implement therapeutic regimens utilizing the drugs described herein as well as different therapeutic regimens using different drugs than those described in the specific examples.

The disclosed techniques allow a user to indicate a subjective insulin need parameter separate from and instead of the user's demographics that is a fixed parameter, such as age, weight, gender, race or the like. The subjective insulin need parameter provides advantages such as 1) it does not significantly increase the burden of insulin therapy for the user of the wearable drug delivery device, 2) it significantly reduces the risk of automated insulin delivery systems, and 3) it allows a simple command from the user to impact automated insulin therapy in real time. The AID algorithm may use the subjective insulin need parameter indicated by the user to specify a subjective coefficient. The subjective coefficient may be used to calculate a specific factor used by the AID algorithm to regulate a user's blood glucose measurement values within range of the user's blood glucose target setting.

The disclosed techniques and devices provide the user with input devices that allow for the input of generalized AID algorithm parameters that are interpretable by the AID algorithm for use in the complex calculations that control a wearable drug delivery device according to the user's diabetes treatment plan.

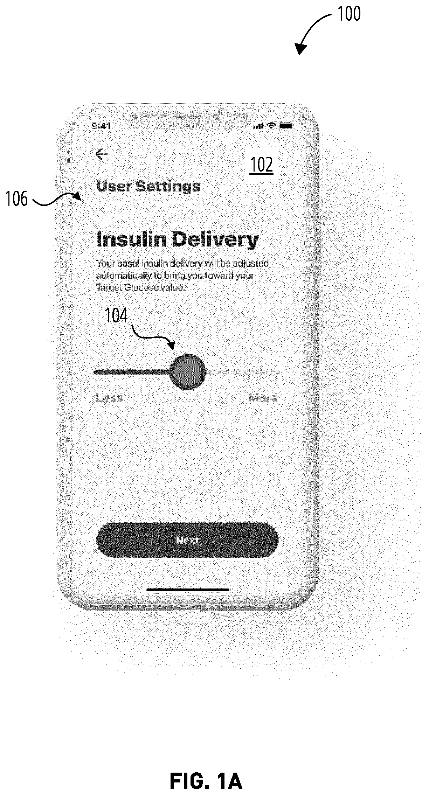

FIG. 1A illustrates an example graphical user interface on a user device according to an embodiment of the disclosed subject matter. The controller 100 may include an input/output device 106, such as a touchscreen display device, that is controllable by a processor executing programming code stored in a memory (shown in a later example). The controller 100 may be operable to present a graphical user interface 102 on the input/output device 106. The graphical user interface 102 may be operable to present screens in which a subjective insulin need parameter may be entered via the user input 104.

The user may be presented with other subjective insulin need parameters or additional subjective insulin need parameters for setting via the user input 104. The subjective insulin need parameter may be labeled according to intuitive impressions instead of specific numerical values (and particularly instead of those specific numerical values that may have to be calculated). Alternatively, some subjective insulin need parameters may be more easily quantified by the user by using relative labels such as "level of activity," more technical aspects, such as "risk tolerance," or more abstract impressions, such as "Insulin Power," or the like. In the FIG. 1A example, the subjective insulin need parameter presented on the graphical user interface 102 for setting is "insulin delivery," which may be a relative label with respect to the present amount of insulin being delivered to a user. However, in some instances, these different labels may all be synonyms for the same subjective insulin need parameter, which may be the "amount of insulin to deliver."

Upon one of these options being presented on the graphical user interface 102, a user may select a subjective level of the subjective insulin need parameter. In an embodiment, the processor may provide one, two, three, or more options that a user can select to indicate their subjective selection. In the example of FIG. 1A, the user may select using user input 104 as part of a continuous, "draggable" level within the range of possible values for the parameter. The user input 104 may be a slider bar as shown in this example.

Of course, other types of user inputs other than user input 104 may be used that enables an input over a range of values. For example, instead of a horizontal slider, a vertical slider bar may be used, a color-coded scheme may be used from green to red or blue to red or the like, a radio wheel, a color palette, keyword inputs via a keyboard or microphone, such as colder or hotter, more or less, or the like.

At least one of the advantages provided by the easy subjective interface is that the AID algorithm is easier to use because is easier for the user to indicate their subjective impressions or feelings instead of entering a string of numbers that are disconnected to what the user is feeling. As a result, the user feels more connected to the wearable drug delivery device and that the AID algorithm is attentive to their condition instead of a programmed device that is receiving the wrong inputs.

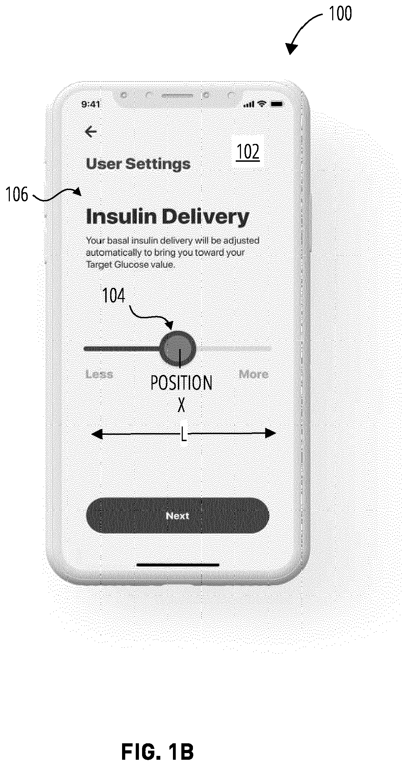

FIG. 1B illustrates an example graphical user interface on a user device according to an embodiment of the disclosed subject matter. FIG. 1B is used to provide an explanation of how the controller responds to the user input 104. The controller 100 may include a processor, an input/output device 106, such as a touchscreen display, and memory. Details of the controller 100 are provided with reference to a later example. The controller 100 may be operable to present a graphical user interface 102 on the input/output device 106. The subjective insulin need parameter may be entered via the user input 104.

The user input 104 is a slider bar, and a range of possible user selections within the input/output device 106. For example, the range of possible user selections may be equal to a number of pixels of the touchscreen display (i.e., input/output device 106) that extend from a first end (e.g., far left) of the slider bar to a second end (e.g., far right). The range of possible user selections may be in inches, millimeters or the like. In the example of FIG. 1B, the user input 104 is at position X. The position X may, for example, be any point within the range of possible user selections.

When considering the user input 104, the starting/default position of the respective subjective insulin need parameter can be set to the "center" of such a range (e.g., at the position of user input 104 shown in FIG. 1B), allowing users to determine both increased or decreased insulin delivery, or at the "top end" (e.g., 600) or the "bottoms end" (e.g., 0) of such a range, allowing users to only determine a level of reduced insulin delivery compared to standard values. The center or X position of the input device in the graphical user interface may represent a default setting at initial onboarding or a previously selected value of the subjective insulin need parameter.

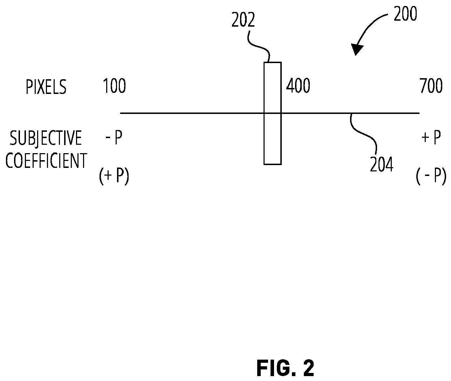

FIG. 2 illustrates a functional illustration of an example user input presented in a graphical user interface. In one embodiment, the AID algorithm may generate a graphical user interface, such as graphical user interface 102 of FIGs. 1A and 1B. The graphical user interface may include a prompt requesting that the user select a subjective insulin need parameter in a graphical user interface presented by a processor executing programming code. The input device 200 is described in more detail with reference to the example of FIG. 2.

The functional input device 200 may include a user input 202 within the total range of distance 204. For example, the total range of distance 204 may be measured in pixels. In this example, the total range of distance 204 may be equal to 600 pixels (i.e., 700 -100 = 600). Each pixel, or a group of pixels (e.g., 10, 25, 200 or the like) may be an increment that may possibly be selected by a user as a user selection. In the example, the first end (e.g., the far left) may be equal to 100 pixels and the second end (e.g., the far right) may be equal to 700. A middle value of the 600 pixel range, such as 400 pixels, may be at the position where the user input 202 is initially located to allow users to indicate their subjective desire of how the user wants the AID algorithm to operate. For example, the input may extend for a range of several hundred pixels either to the left or the right of the current set point (i.e., pixel location 400 may be a default setting at initial onboarding or a previously modified value of this particular subjective insulin need parameter) centered within the total range of distance of the input device in the graphical user interface.

Of course, the number of 600 pixels was selected for ease of discussion, but another number of pixels may be chosen. The range and variety of the impacted parameters can be widened and need not be linked to an individual type of parameter - for instance, certain types of parameters may be allowed to vary bidirectionally (both increase and decrease) while other types of parameters may be limited to only being permitted to decrease or increase.

In the example of FIG. 2, the relationship between the location of the user input 202 along the total range of distance 204 and the subjective coefficient P may be a linear relationship. As shown, the subjective coefficient P may have a value of negative P (i.e., - P) when the user input 202 is positioned at pixel value 100 or may have a value of P (i.e., + P) at pixel value 700. Once a user selection is executed by a user interaction with the user input 202, the actual selected value (in pixels) can be implemented in several areas of both automated and manual pump operations.

In another example, depending upon the subjective insulin need parameter that the user selects to modify, the subjective coefficient P may have a different value when the user input 202 is moved to the left. As shown in parentheses, the subjective coefficient P may be a positive value (i.e., + P) when moved to the left toward the 100 pixel value and may be a negative value (i.e., - P) when moved to the right toward the 700 pixel value.

In an operational example, the AID algorithm may determine which subjective insulin need parameter is being modified by movement of the user input 202, and based on a degree of modification indicated by where the input device 200 is moved within the range of the user input 202 may determine the form of equation and/or the algorithm that is to be used to calculate the subjective coefficient P.

In a general example, the subjective coefficient P may be calculated using a first factor, a second factor, an X value and an L value as follows:

$$P = -[1st\ factor] + (2nd\ factor)\frac{X}{L}$$

Equation 1

where the first factor is a number (such as a decimal value of a percentage) and the second factor is 2 times the first factor, the L value is a range of possible user selections within the input device, which is also referred to as the total range of distance, and the X value is a relative position of the user's selection (i.e., $0 \le X \le L$) via a user input via an input device of a controller. The first factor may be a tunable parameter T that is determined a number of ways. The tunable parameter T may be selected based on a user's age, a user's weight, a user's gender, a type of diabetes, a number of years' experience the user has with automatic drug delivery devices, or the like. In some examples, the second factor may be a multiple of the first factor. In other examples, the second factor may be a set value that is unaffected by the value of the first factor.

Equation 2 shows the replacement of the first factor with tunable parameter T. As shown, the second factor from Equation 1 is replaced with 2T or twice the value of tunable parameter T. After the replacement, the subjective coefficient P may be determined as follows:

$$P = -[T] + (2T)\frac{X}{L}$$

## Equation 2

where T is a tunable parameter, the L value remains a range of possible user selections (e.g., total number of pixels, millimeters, the number of increments, or the like) within the input device, which is also referred to as the total range of distance, and the value X remains a relative position of the user's selection (i.e., $0 \le X \le L$) via the user input within the input device of the controller.

The value of tunable parameter T may select by a healthcare provider, by the AID algorithm, or based on a user input. For example, a healthcare provider may perform a suite of tests on a user to determine clinical factors related to a user's specific diabetes diagnosis, such as HbA1c, glycosylated hemoglobin, blood pressure, lipids and the like, that may be input into the AID algorithm and may be used to establish settings that are used in the control of the user's diabetes. The healthcare provider when considering the results of the tests and the determined clinical factors may make a recommendation for a setting of the tunable parameter T. Alternatively, the AID algorithm may present the user input 202 and use the value associated with the user's input to make settings, such as maximum total daily insulin delivery settings, bolus dosage timing and maximum bolus dosages, drug delivery schedule settings and the like, to the AID algorithm based on the specific factors affected by the value associated with the user input 202.

In an operational example, the factors 0.25 and 0.5 are tunable parameters, where the 0.25 is the first factor and the 0.5 is the second factor that in this example is 2 times the first factor (i.e., 2 time 0.25 = 0.5). It is noted that the subjective coefficient is unitless.

Given the possible variations in the user's daily insulin needs, and the robustness of an AID system to changes in a user's insulin sensitivities, the user's resulting subjective coefficient P can be defined as a range between, for example, -0.25 to 0.25, based on the user's input as:

$$P = -0.25 + 0.5\frac{X}{L}$$

## Equation 3

where X is the relative position of the user's selection within the input device and L is the overall range of possible user selections within the input device.

The values of the subjective coefficient P may increase or decrease setting values associated with the specific factor. Examples of the specific factor may include open loop basal, input basal, glucose target, duration of insulin action, maximum automated insulin delivery limit, or the like.

While open loop basal is a basal rate typically set by the user in open loop operation (i.e., a mode of operation in which the user instructs the system how to operate by inputting a basal rate of insulin delivery), the AID algorithm with sufficient insulin delivery history may be operable to adjust the user's set open loop basal based on the coefficient P. This is in keeping with the advantages of the disclosed subject matter as the user can simply enter the subjective insulin parameter. In an example, the open loop basal may be automatically derived because all the user is inputting is the subjective coefficient. Of course, the user can change the parameter (e.g., the T of Equation 2) in the calculation of the subjective coefficient P over time, if desired.

Alternatively, the AID algorithm may use a user's inputted basal setting to set the user's basal setting for automated mode. However, the AID algorithm may modify the user's inputted basal setting. For example, the modification of the user's inputted basal setting based on CGM values as compared to open loop basal where there is no CGM involvement with the AID algorithm.

In addition, or alternatively, the subjective coefficient P may be used to set the user's glucose target set point (SP), which, for example may be set to 120 mg/dL and further settable in intervals of 10 mg/dL.

Another specific factor that may be managed may be the duration of insulin action (DIA). DIA may be defined as how long insulin takes to act in the body and may be measured in hours, such as 2-8 hours. In this example, DIA is a specific factor that users typically have to input into a device for use by the AID algorithm. DIA impacts insulin onboard (IOB) estimates, and the estimate of IOB is one factor used in automated insulin calculations (e.g., insulin dosage calculations and the like).

Yet another specific factor that may be modified according to the disclosed subject matter may be the maximum automated insulin delivery limit. The maximum automated insulin delivery limit may be based on a lifecycle of a wearable drug delivery device. The lifecycle of a wearable drug delivery device may be approximately 72, 80 or 96 hours or the like. In a typical example, the maximum automated insulin delivery limit may be, for example, approximately 4 times the TDI-based basal delivery maximum. Depending upon different settings and physiological condition data of the user, the maximum automated insulin delivery limit may be changed, for example, to 2 times the TDI-based basal delivery maximum by using a hypoglycemia-protect mode setting of the AID algorithm. In the example, the user's subjective coefficient could impact maximum automated insulin delivery limit setting by, for example, causing changes from 2X to 5X, with a default of 4X, or the like.

In one embodiment, the effect of the subjective coefficient P may mimic pre-existing options to impact insulin delivery, such as temporary basal values, or a combination of settings impacting automated insulin delivery. The subjective coefficient P may be used to impact a wide range of factors, such as in the following examples:

Table 1

| Open loop basal | $b_{OL,f}(t) = (1 + P)b_{OL}(t)$ |
|---|---|
| Input Basal to AID algorithm | $b_{CL,f} = (1 + P)b_{CL}$ |
| Glucose target for AID algorithm | $SP_f(t) = SP(t) - (1 + P)(SP_{max} - SP_{min})$ |
| Duration of Insulin Action | $DIA_f(t) = DIA(t) - (1 + P)(DIA_{m\alpha x} - DIA_{min})$ |
| Maximum automated insulin delivery limit | $U_{Max,f} = (1 + P)U_{Max}$ |

In another embodiment, given the uncertainties in the user's selections and the minor impact of the user's selections, these parameters can instead be varied based on whether the user's selections land in specific ranges. One example of a range based on user selections may be as follows:

Table 2

| User's selection | P< Value 1 | Value 1 ≤ P ≤ Value 2 | Value 2 < P |
|---|---|---|---|
| Open loop basal | $b_{OL,f}(t) = (1 + Pmin)b_{OL}(t)$ | No effect | $b_{OL,f}(t) = (1 + Pmax)b_{OL}(t)$ |
| Input Basal to AID algorithm | $b_{CL,f} = (1 + Pmin)b_{CL}$ | No effect | $b_{CL,f} = (1 + Pmax)b_{CL}$ |
| Glucose target for AID algorithm | $SP_f(t) = SP(t) - (1-P_{min})(SP_{max} - SP_{min})$ | No effect | $SP_f(t) = SP(t) - (1-P_{min})(SP_{max} - SP_{min})$ |
| Duration of Insulin Action | $DIA_f(t) = DIA(t) - (1 +P_{min})(DIA_{max} - DIA_{min})$ | No effect | $DIA_f(t) = DIA(t) - (1 +P_{min})(DIA_{max} - DIA_{min})$ |
| Maximum automated insulin delivery limit | $U_{M\alpha x,f} = (1 + P_{min})U_{Max}$ | No effect | $U_{M\alpha x,f} = (1 + P_{max})U_{Max}$ |

In the example of table 2, the first (left) column may represent a first third of the total range of distance of the user input device, the second (center) column may represent the second third of the total range of distance of the user input device, and the third (right) column may represent the last third of the total range of distance of the user input device. In the example, open loop basal is represented by $b_{OL,f}$, input basal to the AID algorithm is represented by $B_{CL,F}$; Glucose target (i.e., Set point (SP)) for AID algorithm is represented by $SP_f(t)$; Duration of Insulin Action (DIA) is represented by $DIA_f(t)$; and the maximum automated insulin delivery limit is represented by $U_{Max,f}$. In an example, The Value 1 may be equal to $P_{min} + \frac{1}{3}(P_{max} - P_{min})$, the Value 2 may be equal to $P_{max} - \frac{1}{3}(P_{max} - P_{min})$, the value for Pmax may be set by dragging a user input, such as user input 202, all the way to the right, while $P_{min}$ may be set by dragging the user input all the way to the left. To implement the increments of table 2, the range of the user input 202 (which in this case is a slide bar) may be dividing into thirds. In some examples, when the user input indicates that the change is insignificant, such as ±5 of a parameter or within a third of the range as shown in the center column of Table 2, no changes are made because there may not be any or much effect on the user's control outputs.

Referring to the example of FIG. 2, the 600 pixels that form the length of total range of distance 204 may be divided into equal segments of 100 pixels. Of course, in other examples, the segments may be unequal, such as 300, 100, 150 and 50 or other combinations of pixel values that equal 600 pixels. In other examples, the 600 pixels may be coarsely set so the respective values of $P_{min}$ and $P_{max}$ may be 0 and 10, respectively, and P may be any number (or integer) between 0 and 10. In yet other examples, $P_{min}$ and $P_{max}$ may be set to be values related to whichever specific factor is being modified based on the user's input of the subjective insulin need parameter.

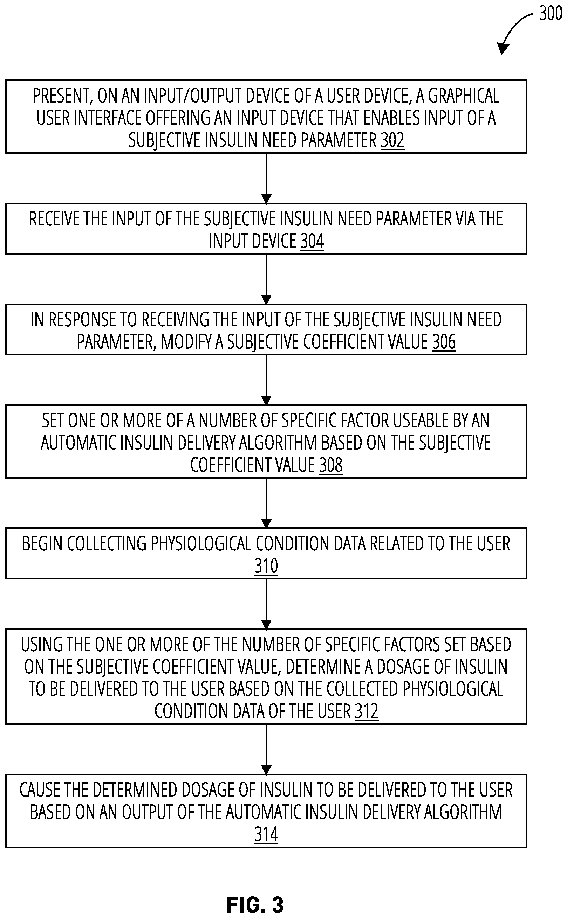

FIG. 3 is a flowchart illustrating a routine in accordance with an embodiment.

In step 302, a processor executing programming code for implementing a method including routine 300 may be operable to present, on a user interface of a user device (shown in a later example), a graphical user interface, such as that shown in earlier examples, offering an input device that enables input of a subjective insulin need parameter.

In step 304, routine 300 receives the input of the subjective insulin need parameter via the input device. The input may be provided using an input device presented on a graphical user interface, such as 102 of FIGs. 1A and 1B. For example, the input may extend for a range of several hundred pixels either to the left or the right of the current set point which may be centered within the total range of distance of the input device in the graphical user interface. Discussion of the input of the subjective insulin need parameter was discussed in detail with reference to FIGs. 1A-2 and such discussion applies to steps 302 and 304.

In step 306, routine 300 in response to receiving the input of the subjective insulin need parameter, modifies a subjective coefficient value. The modification may be an updating the subjective coefficient value that may occur after a period of time.

In an example, the processor prior to modifying the subjective coefficient value, may determine which subjective coefficient corresponds to the subjective insulin need parameter that was presented in the graphical user interface. Based on the determination of which subjective insulin need parameter was presented in the graphical user interface, the processor may determine a form of an equation and/or an algorithm to use to calculate the subjective coefficient. For example, the processor may be operable to select the form of the equation or the algorithm from a look up table, request a download from a cloud service of an algorithm for calculating the subjective coefficient, or the like. The processor may calculate the subjective coefficient using the form of the equation or the algorithm selected from the look up table.

The processor may, for example, be operable to modify the subjective coefficient value by identifying a relative travel distance of the received input in the graphical user interface, determining a ratio of the identified relative distance to a total range of distance that may be traveled, multiplying a modification distance factor by the result of the ratio, and summing the result of the multiplication with a negative modification factor.

In another operational example, the processor may modify the subjective coefficient value or setting by setting the subjective coefficient value at a minimum value for a first predetermined distance of a total range of distance of the user input as the user inputs the subjective insulin need parameter because the subjective insulin need parameter indicated by the movement only effects a minimal change, such as less than or equal to a 5% change in a respective specific factor, such as TDI or the like. As the user input is further moved, the subjective coefficient value may be set to a mid-point of the minimum value and a maximum value after the distance the user input is moved equals or passes the first predetermined distance (in this instance, the inputted distance indicates a subjective insulin need parameter that may affect a change greater than 5% in the respective factor). The subject coefficient value may be increased to the maximum value after the distance traveled by the user input passes or equals a second predetermined distance. This final distance traveled may effect a change in the specific factor greater than the second distance.

In step 308, the processor may set one or more of a number of specific factor useable by an automatic insulin delivery algorithm based on the subjective coefficient value. The number of specific factors may include open loop basal, input basal, glucose target, duration of insulin action, and maximum automated insulin delivery limit.

In step 310, routine 300 begin collecting physiological condition data related to the user. Collection of physiological condition data may be provided by an analyte sensor, such as a continuous glucose monitor (CGM) or the like, that are described with reference other examples. Physiological condition data may be blood glucose measurement values, heart rate, or the like.

In step 312, the processor executing routine 300 may use the one or more of the number of specific factors set based on the subjective coefficient value to determine a dosage of insulin to be delivered to the user based on the collected physiological condition data of the user.

In step 314, routine 300 causes the determined dosage of insulin to be delivered to the user based on an output of the automatic insulin delivery algorithm.

The value of the subjective coefficient may be determining using a number of different implementations given the specific factor being affected by the change in the subjective insulin need parameter input by the user.

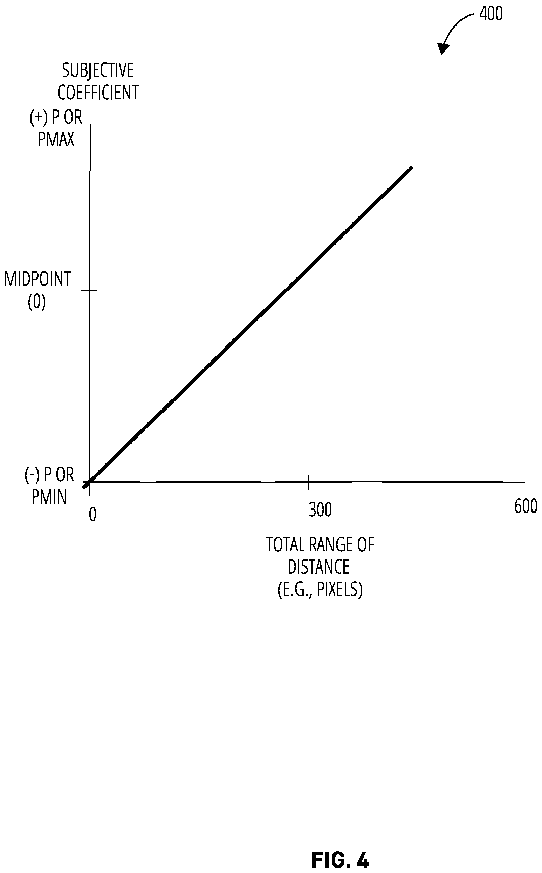

FIG. 4 illustrates a graph illustrating an example for determining a value of a subjective coefficient from a user input in accordance with multiple embodiments.

In the graph 400, the vertical axis is the subjective coefficient (P) values with the value of $P_{min}$ at the origin and $P_{max}$ at the top and the horizontal axis is the total range of distance, which in this example is 600 pixels. The graph 400 utilizes the (-) 0.25 to (+) 0.25 values for the minimum (i.e., $P_{min}$) value and maximum (i.e., $P_{max}$) value, respectively, of the subjective coefficient P from the earlier examples for ease of illustration but other values, such as ±0.5, 0-10 or the like, may also be used. The range of pixels used in the total range of distance of the horizontal axis is 600 pixels as used in the earlier examples.

As shown in the equations 1 and 2 above, the subjective coefficient P may follow a linear curve from $P_{min}$ (i.e., -0.25) to $P_{max}$ (i.e., +0.25). In the linear curve, the center point may be at 0 and 300 pixels.

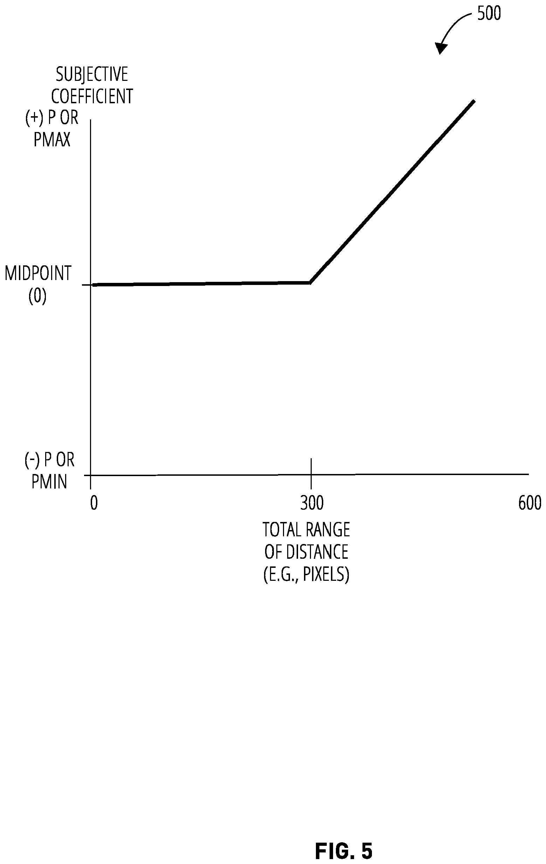

FIG. 5 illustrates a graph illustrating another example for determining a value of a subjective coefficient from a user input in accordance with multiple embodiments.

In the graph 500, the vertical axis is the subjective coefficient (P) values with the value of $P_{min}$ at the origin and $P_{max}$ at the top and the horizontal axis is the total range of distance, which in this example is 600 pixels. The graph 500 utilizes the (-) 0.25 to (+) 0.25 values for the minimum (i.e., $P_{min}$) value and maximum (i.e., $P_{max}$) value, respectively, of the subjective coefficient P from the earlier examples for ease of illustration but other values, such as $\pm 0.5$, 0-10 or the like, may also be used. The range of pixels used in the total range of distance of the horizontal axis is 600 pixels as used in the earlier examples.

In yet another example, an asymmetric curve may be provided that allows the AID algorithm to respond to user inputs that indicate a strong desire for change in the system. In the asymmetric curve example, the subjective coefficient begins changing once a mid-point value (i.e., 300 pixels) of the total range of distance is met (i.e., the asymmetric curve is a straight line or constant at subjective coefficient value 0 from 0 pixels to 300 pixels) and increases as the pixel value extends toward a maximum distance, such as 600 pixels. While the asymmetric curve of graph 500 shows an increase in the subjective coefficient P after the mid-point 300pixel value, the asymmetric curve may be different in different examples. For example, the asymmetric curve may be a straight line that slopes upward from 0 pixels to 300 pixels with a subjective coefficient value that increases from -0.25 to 0, and proceeds as a straight line from 300 pixels to 600 pixels. Of course, other asymmetric curves may be used. For example, the portion extending from 300 pixels to 600 pixels may represent the subjective coefficient (P) value by a utilizing an exponential function, quadratic function, a constant, a logarithmic function or the like. Alternatively, the portion extending from 0 pixels to 300 pixels may represent the subjective coefficient (P) value by a utilizing an exponential function, quadratic function, a constant, a logarithmic function or the like and the portion extending from 300 pixels to 600 pixels may represent the subjective coefficient (P) value by a utilizing a different exponential function, a different linear function, a different quadratic function, a different constant, a different logarithmic function or the like.

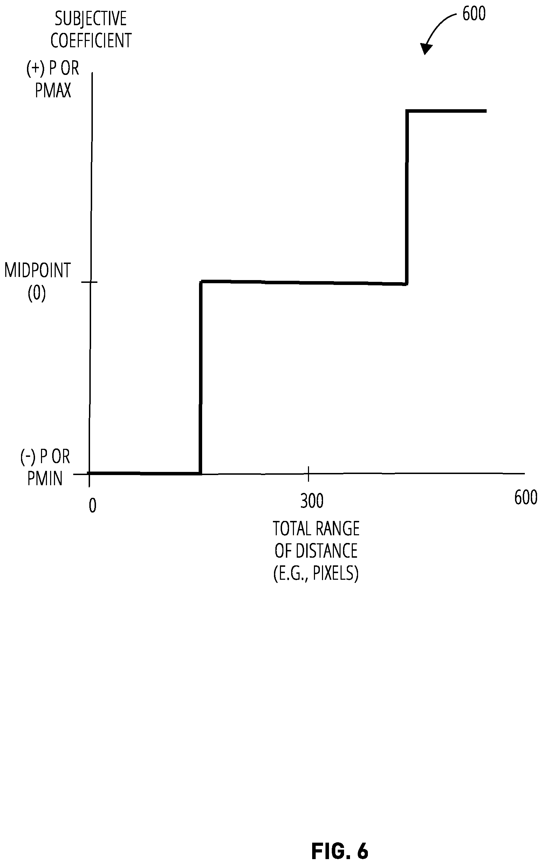

FIG. 6 illustrates a graph illustrating a further different example for determining a value of a subjective coefficient from a user input in accordance with multiple embodiments.

In the graph 600, the vertical axis is the subjective coefficient values with the value of $P_{min}$ at the origin and $P_{max}$ at the top and the horizontal axis is the total range of distance, which in this example is 600 pixels. The graph 600 utilizes the (-) 0.25 to (+) 0.25 values for the minimum (i.e., $P_{min}$) value and maximum (i.e., $P_{max}$) value, respectively, of the subjective coefficient P from the earlier examples for ease of illustration but other values, such as $\pm 0.5$, 0-10 or the like, may also be used. The range of pixels used in the total range of distance of the horizontal axis is 600 pixels as used in the earlier examples.

The graph 600 also shows an example of a stepwise curve that is suitable for implementing the examples discussed with reference to table 2 above. For example, the processor may modify the subjective coefficient value by setting the subjective coefficient value to a minimum value (e.g., $P_{min}$) for a first predetermined distance of a total range of distance of the user input as the user inputs the subjective insulin need parameter. The subjective coefficient value may increase to a value at a mid-point of the minimum value and a maximum value after the distance traversed by the user input equals or passes the first predetermined distance. After the user input travels past or equals a second predetermined distance, the subjective coefficient value may be set to the maximum value (e.g, $P_{max}$).

More specifically, as the user input traverses the total range of distance from 0 pixels to some other number of pixels, such as 150 pixels, the subjective coefficient P value may remain at $P_{min}$ for a first predetermined distance, increases to a mid-point of $P_{min}$ and $P_{max}$ after equaling or passing the first predetermined distance, such as the 150 pixels, and increases again to $P_{max}$ after passing or equaling a second predetermined distance, such as 450 pixels.

Of course, other stepwise curves may be needed depending upon the specific factor for which the subjective coefficient P is being modified. For example, a greater number of steps may be used for input basal or open loop basal rate, or the like.

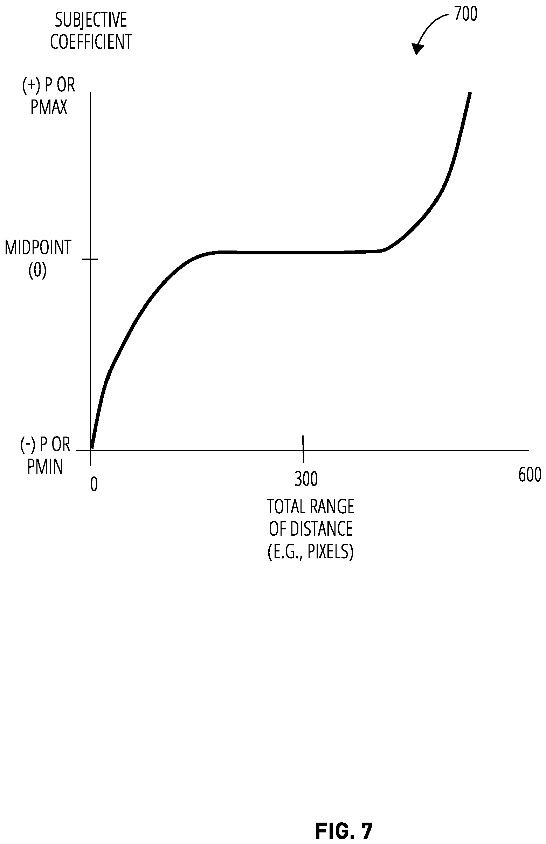

FIG. 7 illustrates a graph illustrating yet another different example for determining a value of a subjective coefficient from a user input in accordance with multiple embodiments.

In the graph 700, the vertical axis is the subjective coefficient values with the value of Pmin at the origin and Pmax at the top and the horizontal axis is the total range of distance, which in this example is 600 pixels. The graph 700 utilizes the (-) 0.25 to (+) 0.25 values for the minimum (i.e., $P_{min}$) value and maximum (i.e., $P_{max}$) value, respectively, of the subjective coefficient P from the earlier examples for ease of illustration but other values, such as $\pm 0.5$, 0-10 or the like, may also be used. The range of pixels used in the total range of distance of the horizontal axis is 600 pixels as used in the earlier examples.

The graph 700 also shows a quadratic curve that provides relative increases in the subjective coefficient P value as the total range of distance increases from 0 pixels to 600 pixels. The example of the quadratic curve shows but one representation of such a curve. The quadratic curve may, for example, have different coefficients and/or constants depending upon the specific factor being modified.

While specific examples were shown in FIGs. 4-7, other examples may also be utilized depending upon the specific factor being addressed or modified.

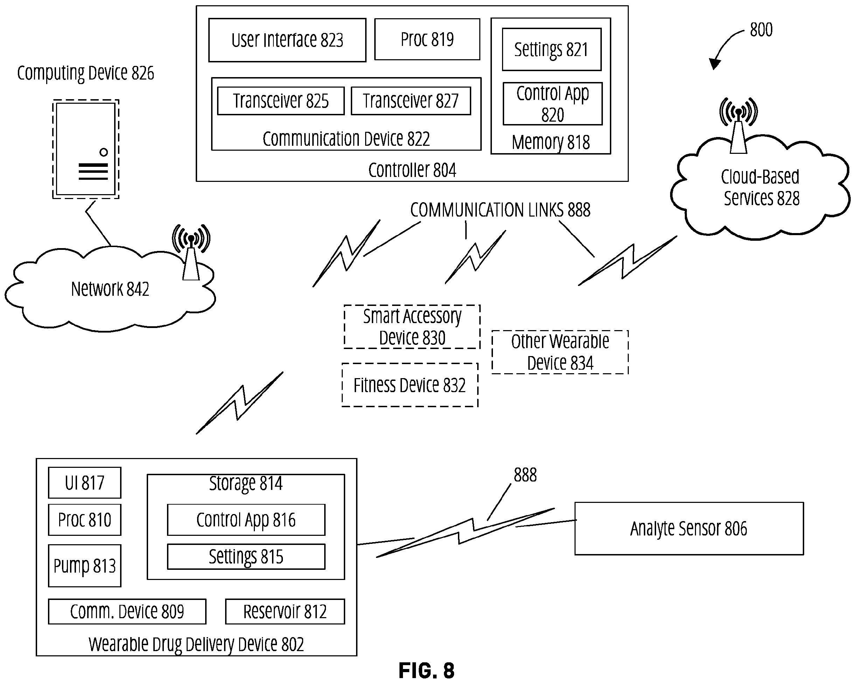

FIG. 8 illustrates a drug delivery system operable to implement the examples of FIGs. 1-7.

In some examples, the drug delivery system 800 is suitable for delivering insulin to a user in accordance with the disclosed embodiments. The drug delivery system 800 may include a wearable drug delivery device 802, a controller 804 and an analyte sensor 806.

The wearable drug delivery device 802 may be a wearable device that is worn on the body of the user. The wearable drug delivery device 802 may be directly coupled to a user (e.g., directly attached to a body part and/or skin of the user via an adhesive, or the like). In an example, a surface of the wearable drug delivery device 802 may include an adhesive to facilitate attachment to the user.

The wearable drug delivery device 802 may include a processor 810. The processor 810 may be implemented in hardware, software, or any combination thereof. The processor 810 may, for example, be a microprocessor, a logic circuit, a field programmable gate array (FPGA), an application specific integrated circuit (ASIC) or a microprocessor coupled to a memory. The processor 810 may maintain a date and time as well as other functions (e.g., calculations or the like). The processor 810 may be operable to execute a control application 816 stored in the storage 814 that enables the processor 810 to direct operation of the wearable drug delivery device 802. The control application 816 may control insulin delivery to the user per an AID control approach as describe herein. For example, the control application 816 may be an AID algorithm. The storage 814 may hold settings 815 for a user, such as specific factor settings, subjective insulin need parameter settings, AID algorithm settings, such as maximum insulin delivery, DIA settings, TDI settings and the like. The analyte sensor 806 may be operable to collect physiological condition data which While not shown, the storage 814 may include both primary memory and secondary memory. The storage 814 may include random access memory (RAM), read only memory (ROM), optical storage, magnetic storage, removable storage media, solid state storage or the like.

The wearable drug delivery device 802 may include a reservoir 812. The reservoir 812 may be operable to store drugs, medications or therapeutic agents suitable for automated delivery, such as insulin, morphine, methadone, hormones, glucagon, glucagon-like peptide, blood pressure medicines, chemotherapy drugs, combinations of drugs, such as insulin and glucagon-like peptide, or the like. A fluid path to the user may be provided via tubing and a needle/cannula (not shown). The fluid path may, for example, include tubing coupling the wearable drug delivery device 802 to the user (e.g., via tubing coupling a needle or cannula to the reservoir 812). The wearable drug delivery device 802 may be operable based on control signals from the processor 810 to expel the drugs, medications or therapeutic agents, such as insulin, from the reservoir 812 to deliver doses of the drugs, medications or therapeutic agents, such as the insulin, to the user via the fluid path.

There may be one or more communications links 888 with one or more devices physically separated from the wearable drug delivery device 802 including, for example, a controller 804 of the user and/or a caregiver of the user and/or a sensor 806. The communication links 888 may include any wired or wireless communication link operating according to any known communications protocol or standard, such as Bluetooth®, Wi-Fi, a near-field communication standard, a cellular standard, or any other wireless protocol

The wearable drug delivery device 802 may also include a user interface 817, such as an integrated display device for displaying information to the user, and in some embodiments, receiving information from the user. For example, the user interface 817 may include a touchscreen and/or one or more input devices, such as buttons, knob or a keyboard that enable a user to provide an input.

In addition, the processor 810 may be operable to receive data or information from the analyte sensor 806 as well as other devices that may be operable to communicate with the wearable drug delivery device 802.

The wearable drug delivery device 802 may interface with a network 842. The network 842 may include a local area network (LAN), a wide area network (WAN) or a combination therein. A computing device 826 may be interfaced with the network, and the computing device may communicate with the insulin delivery device 802. The computing device 826 may be a healthcare provider device through which a user's controller 804 may interact to obtain information, store settings and the like. The AID algorithm operating, as or in cooperation with, the control application 820 may present a graphical user interface on the computing device 826 similar to the graphical user interface 102 of FIGs. 1A and 1B.

The drug delivery system 800 may include an analyte sensor 806 for sensing the levels of one or more analytes of a user. The analyte levels may be used as physiological condition data and be sent to the controller 804 and/or the wearable drug delivery device 802. The sensor 806 may be coupled to the user by, for example, adhesive or the like and may provide information or data on one or more medical conditions and/or physical attributes of the user. The sensor 806 may be a continuous glucose monitor (CGM), or another type of device or sensor that provides blood glucose measurements that is operable to provide blood glucose concentration measurements. The sensor 806 may be physically separate from the wearable drug delivery device 802 or may be an integrated component thereof. The sensor 806 may provide the processor 810 and/or processor 819 with physiological condition data indicative of measured or detected blood glucose levels of the user. The information or data provided by the sensor 806 may be used to adjust drug delivery operations of the wearable drug delivery device 802.

The drug delivery system 800 may also include the controller 804. In the depicted example, the controller 804 may include a processor 819 and a memory 818. The controller 804 may be a special purpose device, such as a dedicated personal diabetes manager (PDM) device. The controller 804 may be a programmed general-purpose device that is a portable electronic device, such as any portable electronic device, smartphone, smartwatch, fitness device, tablet or the like including, for example, a dedicated processor, such as processor, a micro-processor or the like. The controller 804 may be used to program or adjust operation of the wearable drug delivery device 802 and/or the sensor 806. The processor 119 may execute processes to manage a user's blood glucose levels and for control the delivery of the drug or therapeutic agent to the user. The processor 819 may also be operable to execute programming code stored in the memory 818. For example, the memory 818 may be operable to store a control application 820, such as an AID algorithm for execution by the processor 819. The control application 820 may be responsible for controlling the wearable drug delivery device 802, including the automatic delivery of insulin based on recommendations and instructions from the AID algorithm. The

The memory 818 may store the one or more control application 820, settings 821 like those described above for the insulin delivery device 802 and other data and/or programs.

The controller 804 may include a user interface (UI) 823 for communicating with the user. The user interface 823 may include a display, such as a touchscreen, for displaying information. The touchscreen may also be used to receive input when it is a touch screen. The user interface 823 may also include input elements, such as a keyboard, button, knob or the like. In an operation example, the user interface 823 may include a touchscreen display controllable by the processor 819 and be operable to present the graphical user interface, and in response to the received input, the touchscreen display is operable to generate a signal indicative of the subjective insulin need parameter. In an operational example, the user interface may be a touchscreen display controllable by the processor and operable to present a graphical user interface. The touchscreen display, under control of the processor 819, may be operable to, in response to the received input, generate a signal indicative of the subjective insulin need parameter as described with reference to earlier examples.

The controller 804 may interface via a wireless communication link of the wireless communication links 888 with a network, such as a LAN or WAN or combination of such networks that provides one or more servers or cloud-based services 828 via communication device 822. The communication device 822, which may include transceivers 827 and 825, may be coupled to the processor 819. The communication device 822 may be operable to transmit communication signals (e.g., command and control signals) to and receive communication signals (e.g., via transceivers 827 or 825) from the wearable drug delivery device 802 and the analyte sensor 806.

The cloud-based services 828 may be operable to store user history information, such as blood glucose measurement values over a set period of time (e.g., days, months, years), insulin delivery amounts (both basal and bolus dosages), insulin delivery times, types of insulin, indicated meal times, blood glucose measurement value trends or excursions or other user-related diabetes treatment information, specific factor settings including default settings, present settings and past settings, or the like.

Other devices, like smart accessory device 830 (e.g., a smartwatch or the like), fitness device 832 and wearable device 834 may be part of the drug delivery system 800. These devices may communicate with the wearable drug delivery device 802 to receive information and/or issue commands to the wearable drug delivery device 802. These devices 830, 832 and 834 may execute computer programming instructions to perform some of the control functions otherwise performed by processor 810 or processor 819. These devices 830, 832 and 834 may include user interfaces, such as touchscreen displays for displaying information such as current blood glucose level, insulin on board, insulin deliver history, or other parameters or treatment-related information and/or receiving inputs, such as those described with reference to the examples of FIGs. 1A-3. The display may, for example, be operable to present a graphical user interface for providing input, such as request a change in basal insulin dosage or delivery of a bolus of insulin. These devices 830, 832 and 834 may also have wireless communication connections with the sensor 806 to directly receive blood glucose level data or receive in parallel a presentation of the graphical user interface as shown in FIGs. 1A and 1B.

In another operational example, the controller 804 may be operable to execute programming code that causes the processor 819 of the controller 804 to perform the following functions. The processor 819 of the controller 804 may execute an AID algorithm that is one of the control applications 820 stored in the memory or memory 818. The processor may be operable to present, on a user interface that is at least one component of the user interface 823. The user interface 823 may be a touchscreen display controlled by the processor 819, and the user interface 823 is operable to present a graphical user interface that offers an input of a subjective insulin need parameter usable by the AID algorithm. The processor 819 may cause presentation on a user interface of a user device, a graphical user interface offering an input device that enables input of a subjective insulin need parameter. The AID algorithm may generate instructions for the pump 813 to deliver basal insulin to the user or the like.

When an input is received on the user interface 823 in response to presentation of the graphical user interface, the processor 819 may be operable to receive the input of the subjective insulin need parameter via the input device. The processor 819, in response to receiving the input of the subjective insulin need parameter, may be operable to modify a subjective coefficient value, and set one or more of a number of specific factors useable by an automatic insulin delivery algorithm based on the subjective coefficient value.

The processor 819 is also operable to collect physiological condition data related to the user from sensors, such as the analyte sensor 806 or heart rate data from the fitness device 832 or the smart accessory device 830. In an example, the processor 819 executing the AID algorithm may determine a dosage of insulin to be delivered based on the collected physiological condition of the user and a specific factor determined based on the subjective insulin need parameter. The processor 819 may output a control signal via one of the transceivers 827 or 826 to the wearable drug delivery device 802. The outputted signal may cause the processor 810 to deliver command signals to the pump 813 to deliver an amount of related to the determined dosage of insulin in the reservoir 812 to the user based on an output of the AID algorithm.

The various elements of the devices, apparatuses or systems as previously described with reference to FIGs. 1A-3 and 8 may include various hardware elements, software elements, or a combination of both. Examples of hardware elements may include structural members, logic devices, components, processors, microprocessors, circuits, processors, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), memory units, logic gates, registers, semiconductor device, chips, microchips, chip sets, and so forth. Examples of software elements may include software components, programs, applications, computer programs, application programs, system programs, software development programs, machine programs, operating system software, middleware, firmware, software modules, routines, subroutines, functions, methods, procedures, software interfaces, application program interfaces (API), instruction sets, computing code, computer code, code segments, computer code segments, words, values, symbols, or any combination thereof.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, novel subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible considering this disclosure. It is intended that the scope of the present disclosure be limited not by this detailed description, but rather by the claims appended hereto. Future filed applications claiming priority to this application may claim the disclosed subject matter in a different manner and may generally include any set of one or more features as variously disclosed or otherwise demonstrated herein.

Although the present invention is defined in the attached claims, it should be understood that the present invention can also (alternatively) be defined in accordance with the following embodiments:

1. A computing apparatus comprising:

a processor;
a user interface; and
a memory storing instructions and an automatic insulin delivery algorithm that, when executed by the processor, the computing apparatus is operable to:

present, a user input device on a graphical user interface of the user interface, the graphical user interface offering an input device that enables input of a subjective insulin need parameter;
receive the input of the subjective insulin need parameter via the input device;
in response to receiving the input of the subjective insulin need parameter, modify a subjective coefficient value;
set one or more of a number of specific factor useable by an automatic insulin delivery algorithm based on the subjective coefficient value;
begin collect physiological condition data related to a user;
using the one or more of the number of specific factors set based on the subjective coefficient value, determine a dosage of insulin to be delivered to the user based on the collected physiological condition data of the user; and
cause the determined dosage of insulin to be delivered to the user in response to an output of the automatic insulin delivery algorithm.

2. The computing apparatus of embodiment 1, wherein modifying the subjective coefficient value comprises:

identify a relative travel distance of the received input in the graphical user interface;
determine a ratio of the identified relative distance to a total range of distance that may be traveled;
multiply a modification distance factor using the ratio; and
sum a result of the multiplying with a negative modification factor.

3. The computing apparatus of one of the preceding embodiments, wherein modifying the subjective coefficient value comprises:

sets the subjective coefficient value to a minimum value for a first predetermined distance of a total range of distance of the user input as the user inputs the subjective insulin need parameter,
increases the subjective coefficient value to a mid-point of the minimum value and a maximum value after the user input equals or passes the first predetermined distance, and
increases the subjective coefficient value to the maximum value after the user input passes or equals a second predetermined distance.

4. The computing apparatus of one of the preceding embodiments, wherein the specific factor includes open loop basal, input basal, glucose target, duration of insulin action, and maximum automated insulin delivery limit.

5. The computing apparatus of one of the preceding embodiments, wherein the instructions further configure the apparatus to:
update the at least one subjective coefficient after a period of time.

6. The computing apparatus of one of the preceding embodiments, wherein the instructions further configure the apparatus to:

based on the subjective insulin need parameter, determine a form of an equation and/or an algorithm to use to calculate the subjective coefficient;
select the form of the equation or the algorithm from a look up table; and
calculate the subjective coefficient using the form of the equation or the algorithm selected from the look up table.

7. The computing apparatus of one of the preceding embodiments, wherein the user interface is a touchscreen display controllable by the processor and operable to present the graphical user interface, and the touchscreen display is operable to:
in response to the received input, generate a signal indicative of the subjective insulin need parameter.

8. The computing apparatus of one of the preceding embodiments, wherein the computing apparatus further comprises:
a communication device coupled to the processor, wherein the communication device is operable to transmit and receive communication signals from a wearable drug delivery device and an analyte sensor.

9. A method, comprising:

presenting, on a user interface of a user device, a graphical user interface offering an input device that enables input of a subjective insulin need parameter;
receiving the input of the subjective insulin need parameter via the input device;
in response to receiving the input of the subjective insulin need parameter, modifying a subjective coefficient value;
setting one or more of a number of specific factor useable by an automatic insulin delivery algorithm based on the subjective coefficient value;
begin collecting physiological condition data related to a user;
using the one or more of the number of specific factors set based on the subjective coefficient value, determining a dosage of insulin to be delivered to the user based on the collected physiological condition data of the user; and
causing the determined dosage of insulin to be delivered to the user in response to an output of the automatic insulin delivery algorithm.

10. The method of embodiment 9, wherein modifying the subjective coefficient value comprises:

identifying a relative travel distance of the received input in the graphical user interface;
determine a ratio of the identified relative distance to a total range of distance that may be traveled;
multiply a modification distance factor by the ratio; and
sum a result of the multiplying with a negative modification factor.

11. The method of one of embodiments 9 or 10, wherein the number of specific factors includes open loop basal, input basal, glucose target, duration of insulin action, and maximum automated insulin delivery limit.

12. The method of one of embodiments 9 to 11, further comprising:
updating the at least one subjective coefficient after a period of time.

13. The method of one of embodiments 9 to 12, further comprising:

based on the subjective insulin need parameter, determining a form of an equation and/or an algorithm to use to calculate the subjective coefficient;
selecting the form of the equation or the algorithm from a look up table; and
calculating the subjective coefficient using the form of the equation or the algorithm selected from the look up table.

14. The method of one of embodiments 9 to 13, wherein modifying the subjective coefficient value comprises:

setting the subjective coefficient value at a minimum value for a first predetermined distance of a total range of distance of the user input as the user inputs the subjective insulin need parameter,
increasing the subjective coefficient value to a mid-point of the minimum value and a maximum value after the user input equals or passes the first predetermined distance, and
increases the subjective coefficient value to the maximum value after the user input passes or equals a second predetermined distance.

15. A non-transitory computer-readable storage medium, the computer-readable storage medium including instructions that when executed by a processor, cause the processor to:

present, on a user interface of a user device, a graphical user interface offering an input device that enables input of a subjective insulin need parameter;
receive the input of the subjective insulin need parameter via the input device;
in response to receiving the input of the subjective insulin need parameter, modifying a subjective coefficient value;
set one or more of a number of specific factor useable by an automatic insulin delivery algorithm based on the subjective coefficient value;
begin collect physiological condition data related to a user;
using the one or more of the number of specific factors set based on the subjective coefficient value, determine a dosage of insulin to be delivered to the user based on the collected physiological condition data of the user; and
cause the determined dosage of insulin to be delivered to the user in response to an output of the automatic insulin delivery algorithm.

16. The computer-readable storage medium of embodiment 15, wherein when modifying the subjective coefficient value, the processor is further configured to:

identify a relative travel distance of the received input in the graphical user interface;
determine a ratio of the identified relative distance to a total range of distance that may be traveled;
multiply a modification distance factor by the ratio; and
sum the result of the multiplying with a negative modification factor.

17. The computer-readable storage medium of one of embodiments 15 or 16, wherein when modifying the subjective coefficient value, the processor is further configured to:

set the subjective coefficient value at a minimum value for a first predetermined distance of a total range of distance of the user input as the user inputs the subjective insulin need parameter,
increase the subjective coefficient value to a mid-point of the minimum value and a maximum value after a distance traveled by the user input equals or passes the first predetermined distance, and
increases the subjective coefficient value to the maximum value after a farther distance traveled the user input passes or equals a second predetermined distance.

18. The computer-readable storage medium of one of embodiments 15 to 17, wherein the number of specific factors includes open loop basal, input basal, glucose target, duration of insulin action, and maximum automated insulin delivery limit.

19. The computer-readable storage medium of one of embodiments 15 to 18, wherein the instructions when executed by the processor further cause the processor to:
update the at least one subjective coefficient after a period of time.

20. The computer-readable storage medium of one of embodiments 15 to 19, wherein the instructions when executed by the processor further cause the processor to:

based on the subjective insulin need parameter, determine a form of an equation and/or an algorithm to use to calculate the subjective coefficient;
select the form of the equation or the algorithm from a look up table; and
calculate the subjective coefficient using the form of the equation or the algorithm selected from the look up table.

**Claims**

1. A computing apparatus comprising:

   a processor;
   a user interface; and
   a memory storing instructions and an automatic insulin delivery algorithm that, when executed by the processor, the computing apparatus is operable to:

      present, a user input device on a graphical user interface of the user interface, the graphical user interface offering an input device that enables input of a subjective insulin need parameter;
      receive the input of the subjective insulin need parameter via the input device;
      in response to receiving the input of the subjective insulin need parameter, modify a subjective coefficient value;
      set one or more of a number of specific factor useable by an automatic insulin delivery algorithm based on the subjective coefficient value;
      begin collect physiological condition data related to a user;
      using the one or more of the number of specific factors set based on the subjective coefficient value, determine a dosage of insulin to be delivered to the user based on the collected physiological condition data of the user; and
      cause the determined dosage of insulin to be delivered to the user in response to an output of the automatic insulin delivery algorithm.

2. The computing apparatus of claim 1, wherein modifying the subjective coefficient value comprises:

   identify a relative travel distance of the received input in the graphical user interface;
   determine a ratio of the identified relative distance to a total range of distance that may be traveled;
   multiply a modification distance factor using the ratio; and
   sum a result of the multiplying with a negative modification factor.

3. The computing apparatus of one of the preceding claims, wherein modifying the subjective coefficient value comprises:

   sets the subjective coefficient value to a minimum value for a first predetermined distance of a total range of distance of the user input as the user inputs the subjective insulin need parameter,
   increases the subjective coefficient value to a mid-point of the minimum value and a maximum value after the user input equals or passes the first predetermined distance, and
   increases the subjective coefficient value to the maximum value after the user input passes or equals a second predetermined distance.

4. The computing apparatus of one of the preceding claims, wherein the specific factor includes open loop basal, input basal, glucose target, duration of insulin action, and maximum automated insulin delivery limit.

5. The computing apparatus of one of the preceding claims, wherein the instructions further configure the apparatus to:
   update the at least one subjective coefficient after a period of time.

6. The computing apparatus of one of the preceding claims, wherein the instructions further configure the apparatus to:

   based on the subjective insulin need parameter, determine a form of an equation and/or an algorithm to use to calculate the subjective coefficient;
   select the form of the equation or the algorithm from a look up table; and
   calculate the subjective coefficient using the form of the equation or the algorithm selected from the look up table.

7. The computing apparatus of one of the preceding claims, wherein the user interface is a touchscreen display controllable by the processor and operable to present the graphical user interface, and the touchscreen display is operable to:
   in response to the received input, generate a signal indicative of the subjective insulin need parameter.

8. The computing apparatus of one of the preceding claims, wherein the computing apparatus further comprises:
a communication device coupled to the processor, wherein the communication device is operable to transmit and receive communication signals from a wearable drug delivery device and an analyte sensor.

9. A method, comprising:

presenting, on a user interface of a user device, a graphical user interface offering an input device that enables input of a subjective insulin need parameter;
receiving the input of the subjective insulin need parameter via the input device;
in response to receiving the input of the subjective insulin need parameter, modifying a subjective coefficient value;
setting one or more of a number of specific factor useable by an automatic insulin delivery algorithm based on the subjective coefficient value;
begin collecting physiological condition data related to a user;
using the one or more of the number of specific factors set based on the subjective coefficient value, determining a dosage of insulin to be delivered to the user based on the collected physiological condition data of the user; and
causing the determined dosage of insulin to be delivered to the user in response to an output of the automatic insulin delivery algorithm.

10. A non-transitory computer-readable storage medium, the computer-readable storage medium including instructions that when executed by a processor, cause the processor to:

present, on a user interface of a user device, a graphical user interface offering an input device that enables input of a subjective insulin need parameter;
receive the input of the subjective insulin need parameter via the input device;
in response to receiving the input of the subjective insulin need parameter, modifying a subjective coefficient value;
set one or more of a number of specific factor useable by an automatic insulin delivery algorithm based on the subjective coefficient value;
begin collect physiological condition data related to a user;
using the one or more of the number of specific factors set based on the subjective coefficient value, determine a dosage of insulin to be delivered to the user based on the collected physiological condition data of the user; and
cause the determined dosage of insulin to be delivered to the user in response to an output of the automatic insulin delivery algorithm.

11. The computer-readable storage medium of claim 10, wherein when modifying the subjective coefficient value, the processor is further configured to:

identify a relative travel distance of the received input in the graphical user interface;
determine a ratio of the identified relative distance to a total range of distance that may be traveled;
multiply a modification distance factor by the ratio; and
sum the result of the multiplying with a negative modification factor.

12. The computer-readable storage medium of one of claims 10 or 11, wherein when modifying the subjective coefficient value, the processor is further configured to:

set the subjective coefficient value at a minimum value for a first predetermined distance of a total range of distance of the user input as the user inputs the subjective insulin need parameter,
increase the subjective coefficient value to a mid-point of the minimum value and a maximum value after a distance traveled by the user input equals or passes the first predetermined distance, and
increases the subjective coefficient value to the maximum value after a farther distance traveled the user input passes or equals a second predetermined distance.

13. The computer-readable storage medium of one of claims 10 to 12, wherein the number of specific factors includes open loop basal, input basal, glucose target, duration of insulin action, and maximum automated insulin delivery limit.

14. The computer-readable storage medium of one of claims 10 to 13, wherein the instructions when executed by the processor further cause the processor to:

update the at least one subjective coefficient after a period of time.

**15.** The computer-readable storage medium of one of claims 10 to 14, wherein the instructions when executed by the processor further cause the processor to:

based on the subjective insulin need parameter, determine a form of an equation and/or an algorithm to use to calculate the subjective coefficient;
select the form of the equation or the algorithm from a look up table; and
calculate the subjective coefficient using the form of the equation or the algorithm selected from the look up table.

FIG. 1A

**FIG. 1B**

PIXELS    100                          400                700

SUBJECTIVE    - P                                          + P
COEFFICIENT
              (+ P)                                        ( - P)

202    200    204

**FIG. 2**

300

PRESENT, ON AN INPUT/OUTPUT DEVICE OF A USER DEVICE, A GRAPHICAL USER INTERFACE OFFERING AN INPUT DEVICE THAT ENABLES INPUT OF A SUBJECTIVE INSULIN NEED PARAMETER 302

RECEIVE THE INPUT OF THE SUBJECTIVE INSULIN NEED PARAMETER VIA THE INPUT DEVICE 304

IN RESPONSE TO RECEIVING THE INPUT OF THE SUBJECTIVE INSULIN NEED PARAMETER, MODIFY A SUBJECTIVE COEFFICIENT VALUE 306

SET ONE OR MORE OF A NUMBER OF SPECIFIC FACTOR USEABLE BY AN AUTOMATIC INSULIN DELIVERY ALGORITHM BASED ON THE SUBJECTIVE COEFFICIENT VALUE 308

BEGIN COLLECTING PHYSIOLOGICAL CONDITION DATA RELATED TO THE USER 310

USING THE ONE OR MORE OF THE NUMBER OF SPECIFIC FACTORS SET BASED ON THE SUBJECTIVE COEFFICIENT VALUE, DETERMINE A DOSAGE OF INSULIN TO BE DELIVERED TO THE USER BASED ON THE COLLECTED PHYSIOLOGICAL CONDITION DATA OF THE USER 312

CAUSE THE DETERMINED DOSAGE OF INSULIN TO BE DELIVERED TO THE USER BASED ON AN OUTPUT OF THE AUTOMATIC INSULIN DELIVERY ALGORITHM 314

**FIG. 3**

400

SUBJECTIVE
COEFFICIENT

(+) P OR
PMAX

MIDPOINT
(0)

(-) P OR
PMIN

0          300          600

TOTAL RANGE OF
DISTANCE
(E.G., PIXELS)

**FIG. 4**

500

SUBJECTIVE
COEFFICIENT
(+) P OR
PMAX

MIDPOINT
(0)

(-) P OR
PMIN

0                           300                          600

TOTAL RANGE
OF DISTANCE
(E.G., PIXELS)

**FIG. 5**

**FIG. 6**

FIG. 7

**FIG. 8**

EP 4 216 227 A1

Europäisches
Patentamt

European
Patent Office

Office européen
des brevets

# EUROPEAN SEARCH REPORT

**Application Number**

EP 23 15 2848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/386936 A1 (SJOLUND JOHN [US] ET AL) 16 December 2021 (2021-12-16) * The whole document, in particular: Paragraphs: [0033], [0060], [0089], [0138], [0146], [0175]. Figures 7, 24, 26, 33. * ----- | 1-15 | INV. G16H20/17 G16H40/63 G06F3/04847 |
| X | Birkholz Jeanette: "Erfahrungsbericht zur CamAPS FX App – Mediq Direkt Diabetes", , 22 February 2021 (2021-02-22), pages 1-29, XP093039452, Retrieved from the Internet: URL:https://www.mediqdirekt.de/blog/cam-aps-fx-app-erfahrungsbericht [retrieved on 2023-04-17] * The whole document, in particular: Sections "Boost function" and "Ease-off function". Figure 2. * ----- | 1-15 | |
| X | Birkholz Jeannette: "Erfahrungsbericht zur CamAPS FX App – Mediq Direkt Diabetes", , 10 April 2021 (2021-04-10), pages 1-14, XP093039455, Retrieved from the Internet: URL:https://web.archive.org/web/20210410210613/https://www.mediqdirekt.de/blog/cam-aps-fx-app-erfahrungsbericht/ [retrieved on 2023-04-17] * The whole document, in particular: Sections "Boost function" and "Ease-off function". Figure 2. * ----- -/-- | 1-15 | TECHNICAL FIELDS SEARCHED (IPC) G16H |

The present search report has been drawn up for all claims

| Place of search | Date of completion of the search | Examiner |
|---|---|---|
| Munich | 21 April 2023 | Zacharias, Malte |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)

**EUROPEAN SEARCH REPORT**

Application Number

EP 23 15 2848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 6 157 381 A (BATES CARY L [US] ET AL) 5 December 2000 (2000-12-05) * The whole document, in particular: Section "Summary of the invention". Figure 17. * | 2,3,6, 11,12,15 | |
| A | US 2008/184167 A1 (BERRILL ARTHUR R [CA] ET AL) 31 July 2008 (2008-07-31) * The whole document, in particular: Figure 2. * | 2,3,6, 11,12,15 | |
| A | US 2018/107367 A1 (RINNEBERG THOMAS [DE] ET AL) 19 April 2018 (2018-04-19) * The whole document, in particular: Paragraph [0111]. Figure 5. * | 2,3,6, 11,12,15 | |
| A | WO 2020/219619 A1 (PROGENICS PHARM INC [US]) 29 October 2020 (2020-10-29) * The whole document, in particular: Figures 6 and 7. * | 2,3,6, 11,12,15 | |

TECHNICAL FIELDS SEARCHED (IPC)

The present search report has been drawn up for all claims

| Place of search | Date of completion of the search | Examiner |
|---|---|---|
| Munich | 21 April 2023 | Zacharias, Malte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 2848

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2023

| Patent document cited in search report | | Publication date | Patent family member(s) | | Publication date |
|---|---|---|---|---|---|
| US 2021386936 | A1 | 16-12-2021 | US 2019184108 | A1 | 20-06-2019 |
| | | | US 2021386936 | A1 | 16-12-2021 |
| US 6157381 | A | 05-12-2000 | NONE | | |
| US 2008184167 | A1 | 31-07-2008 | EP 1950652 | A1 | 30-07-2008 |
| | | | US 2008184167 | A1 | 31-07-2008 |
| US 2018107367 | A1 | 19-04-2018 | NONE | | |
| WO 2020219619 | A1 | 29-10-2020 | AU 2020263381 | A1 | 14-10-2021 |
| | | | CA 3134745 | A1 | 29-10-2020 |
| | | | CN 113748443 | A | 03-12-2021 |
| | | | EP 3959683 | A1 | 02-03-2022 |
| | | | JP 2022530040 | A | 27-06-2022 |
| | | | US 2022180487 | A1 | 09-06-2022 |
| | | | WO 2020219619 | A1 | 29-10-2020 |

EPO FORM P0459

For more details about this annex : see Official Journal of the European Patent Office, No. 12/82